# EUROPEAN PATENT SPECIFICATION

(11) **EP 1 214 017 B1**
(45) Date of publication and mention of the grant of the patent: **12.08.2009**
(21) Application number: 00942830.1
(22) Date of filing: 15.06.2000
(51) Int. Cl.: A61F 2/06

(54) **DOUBLE LAYER INTRALUMINAL GRAFT**
INTRALUMINALES DOPPELSCHICHTTRANSPLANTAT
GREFFE INTRALUMINALE A COUCHE DOUBLE

(30) Priority: 23.09.1999 AU PQ302799; 29.12.1999 US 473618
(43) Date of publication of application: 19.06.2002
(73) Proprietor: Endogad Research Pty Limited, Parramatta, NSW 2150 (AU)
(72) Inventor: DEHDASHTIAN, Mark, Costa Mesa, CA 92626 (US); WHITE, Geoffrey, H., East Balmain, New South Wales 2041 (AU); YU, Weiyun, Birchgrove, New South Wales 2041 (AU)
(74) Representative: Baker, Colin John
(86) International application number: PCT/US2000/016457
(87) International publication number: WO 2001/021102

(56) References cited:
- WO-A-00/38590
- WO-A-00/45742
- US-A- 5 639 278
- US-A- 5 662 675
- US-A- 5 843 160
- US-A- 5 871 538
- US-A- 6 010 529
- US-A- 6 045 557

## Description

### Field of the Invention

The present invention relates to an intraluminal device for use in the treatment of aneurysmal or stenotic disease.

### Background of the Invention

It is known to use intraluminal grafts and stents of various designs for the treatment of aneurysms such as aortic aneurysms and occlusive diseases affecting the vasculature or other vessels comprising, inter alia, the hepatobiliary and genito-urinary tracts (which are all hereinafter "vessels"). It is known to form such an intraluminal device of a sleeve in which is disposed a plurality of self-expanding wire stents (see Balko A. et al (1986) Transfemoral Placement of Intraluminal Polyurethane Prosthesis for Abdominal Aortic Aneurysms, 40 Journal of Surgical Research 40, 305-309; Mirich D. et al. (1989) Percutaneously Placed Endovascular Grafts for Aortic Aneurysms: Feasibility Study 170(3) Radiology 170(3), 1033-1037).

In the past, such devices have commonly been used in the treatment of aneurysms, see for example, US Letters Patents Numbers 5782904, 5968068, 5976192, 6013092, and 6 110 191 all subject to assignment to the entity owning all rights in the instant subject matter. However, it has been recognized that it is within the ambit of some such devices that they also be used to treat stenotic lesions. Whatever the purpose for which an intraluminal device is being used, it has the capacity to be inserted percutaneously through a distal (or proximal) and connecting vessel to that in which the device is to be used. For example, the device may be inserted through the femoral artery in a catheter, where the device is intended to be used in the treatment of a lesion within the aorta. Upon release of the device from the catheter it may expand to a desirable size, and may extend above and below the lesion thereby bridging that lesion. This method of inserting the device into the body of a patient is applicable in the treatment of aneurysmal disease or stenotic disease.

Further, where the device is used in the treatment of an aneurysm which extends from a single vessel into one or more divergent vessels, a bifurcated or "trouser graft" is required as described in, for example, US patent Nos. 2006/001576, 6451053, 6565596, 6689158 and 6685736 each of which is subject to assignment to a common entity. Other known devices include US 5662675, which discloses a modular system for forming a bifurcated intraluminal graft from a series of individual components to repair defective body lumen. US 5639278 discloses an endoluminal graft suitable for use in a bifurcated body vessel location comprising a tubular support having both a liner and a cover.

There may be a number of problems associated with such known intraluminal devices which may include rupture of the intraluminal graft due to general wear or damage upon insertion into the vessel. While thicker and more durable grafts may be designed to overcome this problem, such grafts in turn require a larger sized catheter for delivery into the affected vessel. The limitation in this regard is the size of the artery in which the catheter is being inserted. For example in the situation where a graft is inserted to bridge an aneurysm in the thoracic aorta, the catheter bearing the graft must be inserted through one of the femoral arteries, moved through the femoral artery, into the common iliac artery and eventually into the aorta. If the catheter is too large in diameter, it is not suitable for insertion into the femoral artery of a patient.

Further, in so-called "trouser grafts", the graft may have a tendency to "kink" in an area of the graft immediately above the area of bifurcation. Whilst kinking in this region may be overcome by adding further reinforcing wires integral the material of the graft in this region, this may increase the diameter of the graft and thus a larger size of catheter may be required to introduce the graft into the vessel.

The present invention is directed to an alternative form of intraluminal device which in preferred forms may overcome the above problems and in fact has been revised and novel enhanced iterations advanced to the market for the first time post-1999. Likewise further embodiments are yet to be released.

### Summary of the Invention

In the first aspect, the present invention consists in an intraluminal device, as defined in independent claim 1, comprising a first tubular graft body and at least a second tubular graft body, each tubular graft body having a length and a first and at least second end wherein when the intraluminal device is disposed within a vessel of a patient, a majority of the length of the second tubular graft body overlaps with a majority of the length of the first tubular graft body.

In another aspect, not of this invention consists in a method for positioning a first and at least a second tubular graft segment, cuff, or body in a vessel of a patient's body, the method including the steps of introducing a catheter into the vessel in the body, causing the first tubular graft member to be moved through the catheter until it extends into the vessel from the proximal end of the catheter, urging the first tubular graft body into contact with the wall of the vessel; causing the second tubular graft member to be moved through the catheter until a substantial length of the second tubular graft body overlaps a substantial length of the first tubular graft body and urging the second tubular graft body into contact with the first tubular graft body.

For example, the first tubular graft body is moved through the catheter on an inflatable balloon until it extends into the vessel from the proximal end of the catheter. The balloon is then inflated to cause the first tubular graft body to be urged into contact with the wall of the vessel and subsequently deflated and withdrawn from the vessel. The second tubular graft body is then moved through the catheter on an inflatable balloon until a substantial length of the second tubular graft body overlaps a substantial length of the second tubular graft body and the balloon inflated such that the second tubular graft body is urged into contact with the first tubular graft body. The balloon is then deflated and the catheter withdrawn from the vessel. Alternatively, the first tubular graft body is moved through the catheter until it extends into the vessel. A balloon may then be passed through the catheter until it extends from the proximal end of the catheter internal the first tubular graft body whereupon the balloon is inflated to cause the first tubular graft body to be urged into contact with the wall of the vessel. The second tubular graft body may be similarly introduced into the vessel.

In another configuration, in place of a balloon, the first and the second tubular graft bodies may be self expandable such that when the tubular graft body extends from the proximal end of the catheter it takes on an expanded configuration such that it is caused to contact the vessel wall.

In examples, the first and the second tubular graft bodies have an equal cross sectional area or a different cross sectional area before insertion into the vessel of a patient. When *in situ*, however, typically the maximum cross sectional area of the first tubular graft is greater than the maximum cross sectional area of the second tubular graft body. Accordingly, the first tubular graft body is inserted into the vessel of a patient and the second tubular graft body inserted internal the first tubular graft body such that a substantial portion of the second tubular graft body overlaps with a substantial portion of the first tubular graft body. Alternatively, the maximum cross sectional area of the first tubular graft body may be less than the maximum cross sectional area of the second tubular graft body such that upon placement of the first tubular graft body within the vessel of a patient, the second tubular graft body is introduced such that the second tubular graft body is placed external to the first tubular graft body.

In one illustrated configuration, the entire length of the second tubular graft body is overlapped with a length of the first tubular graft body.

In a further illustrated configuration the entire length of the first tubular graft body is overlapped with a length of the second tubular graft body.

In yet a further illustrated configuration the first and second tubular graft bodies are of the same length and the entire length of the second tubular graft body is overlapped with the entire length of the first tubular graft body.

Prototypes of related iterations have been used and tested. According to yet a still further configuration, the area of overlap of the two tubular graft bodies is greater than 50% of the length of the tubular graft bodies. More preferably, the area of overlap is greater than 75-80% and more preferably still between 80 and 100%.

In a still further configuration, a portion of the second tubular graft body does not overlap with the first tubular graft body, said non-overlapping portion extending longitudinally from the first tubular graft member into the lumen of the vessel in which the device is disposed.

In one tested and working configuration, the first and at least second tubular graft bodies are circumferentially reinforced along their length by a plurality of separate, spaced apart, malleable wires. Each of the wires can have a generally closed sinusoidal shape.

In still a further configuration, the first and second tubular graft bodies are longitudinally reinforced along their length by a longitudinally reinforcing malleable wire. The longitudinally reinforcing wire may be positioned between two circumferentially reinforcing wires. Several longitudinally reinforcing wires may be positioned along the length of both the first and the second tubular graft bodies. Each wire may be generally straight in shape or may have a zig-zag or sinusoidal shape. The presence of a longitudinally reinforcing wire has the advantage of reinforcing the tubular graft bodies such that neither tubular graft body is forced into a compressed state along its longitudinal axis.

In a still further configuration, one of the tubular graft bodies (for example, the first) may be longitudinally reinforced, said tubular graft body having no circumferential reinforcement. In this configuration the other (for example, the second) tubular graft body is circumferentially reinforced, said other tubular graft body having no longitudinal reinforcement.

In another alternate configuration, the first and at least second tubular graft bodies are circumferentially reinforced along their length by one continuous wire, the wire taking on a spiral configuration along the length of both the first and at least second tubular graft bodies. Likewise, helices in the configuration of the wire are within the scope of the instant techniques.

In another configuration the first and at least second tubular graft bodies are circumferentially reinforced along their length by a series of wires or one continuous wire woven into the material of the tubular graft bodies such that the wires are not exposed at either the outer or the inner surface of the tubular graft bodies. Such an enclosed wireform arrangement is particularly useful in a configuration wherein the tubular graft body is inserted into the vessel of a patient and caused to expand within the vessel of the patient by way of an inflatable balloon. In a further configuration wherein the tubular graft body is adapted to self expand without the need for a balloon it is not required that the wires be entirely enclosed and indeed it is preferred that at least a portion of the wires are positioned on either the outside or the inside surfaces of the tubular graft bodies. Like wise, it is known to artisans that, for example, any techniques in the commonly owned or assigned patents and patent applications such as interweaving of wireforms having predetermined or pre-ordained spatial orientations made of, for example, Elgiloy ^{®} wireforms with Dacron ^{®} grafts (available from Baxter Vascular Systems Division, Irvine, California) or a PTFE (Baxter Healthcare Corporation, Laguna Hills, California) and Nitinol^{™} (Memry Metal, California) are equally applicable and work well within human patients.

In a further configuration, both tubular graft bodies are either balloon expandable or self expandable. Alternatively, one of the tubular graft bodies may be balloon expandable and the other tubular graft body self expandable. Preferably, when in situ within the vessel of a patient the outer tubular graft body (for example, the first tubular graft body) which is in contact with the vessel wall is of the self expandable type and the inner tubular graft body (for example the second tubular graft body) is of the balloon expandable type.

In a still further configuration at least a portion of the length of one tubular graft body may be adapted to be balloon expandable and the remaining length of the same tubular graft body adapted to be self expandable. In a particularly preferred embodiment, the first end of the tubular graft body is self expandable and the remainder of the tubular graft body is balloon expandable. This embodiment is of particular significance when it is understood that misplacing of a balloon internal the tubular graft body such that the balloon extends past the first end of the tubular graft body may cause inflation of the vessel wall itself and may potentially result in rupture of the vessel wall. With the first end of the tubular graft body adapted such that it is self expandable, the balloon need not be inserted as far towards the first end thereby reducing the risk of over extension of the balloon and subsequent damage of the vessel wall.

Also described herein, the first and at least second tubular graft bodies are reinforced along their length by a series of separate spaced apart stents. Alternatively at least some of the stents may be interconnected or all of the stents may be interconnected to form one continuous stent.

Also described herein, the first tubular graft body includes at least one first engagement member which is connected to or integral with a wall of the first tubular graft body at a position intermediate the ends of the first tubular graft body.

Also described herein, the at least one first engagement member is adapted to extend externally of the wall of the first tubular graft body. When disposed in a vessel, the engagement member preferably abuts the surrounding vessel wall thereby securing the first tubular graft body within the vessel.

Also described herein, the at least one first engagement member comprises the ends of the malleable wires which are joined together to form a tail means. Each tail means is preferably on the outside of the graft body and positioned to lie along its radially outer surface. The ends may be joined by welding, by being twisted together or in any other suitable manner. The ends of adjacent wires preferably project in generally opposite directions along the first tubular graft body and when the first tubular graft body is inserted into a vessel those wires that engage the vessel wall will assist in preventing dislodgement of the first tubular graft body within the vessel.

Also described herein, several of the tails of the malleable wires may be on or adjacent an inside wall of the first tubular graft body such that upon insertion of the second tubular graft body internal the first tubular graft body, the tails engage with the wall of the second tubular graft body thereby securing the second tubular graft body in place. Frictional, mechanical, and frustoconical means for engaging are further used with the overlapping aspects.

Also described herein the at least one first engagement member comprises a hook-like member adapted to project from at least the first end of the first tubular graft body, when disposed in a vessel, the hook-like member preferably engages the wall of the vessel in which the first tubular graft body is disposed thereby preventing dislodgement of the first tubular graft body within the vessel. Alternatively, the first tubular graft body includes a stent or a series of spaced apart stents which form a framework to which may be attached an endoluminal graft. Expansion of the stent or stents will cause the first tubular graft body to expand and press against the wall of a vessel into which it has been placed thereby securing the first tubular graft body in that vessel.

Also described herein, the first tubular graft body includes at least one second engagement member positioned intermediate the two ends of the first tubular graft body, the at least one second engagement member is adapted to project into the lumen of the first tubular graft body such that it engages the wall of the second tubular graft body.

The at least one second engagement member preferably includes a ring-like member adapted to project into the lumen of the first tubular graft body from the inner surface of the first tubular graft body.

Furthermore, the ring-like member can be continuous or discontinuous in configuration.

Alternatively, the second engagement member could include hook-like members circumferentially disposed around the inner surface of the first tubular graft body. In another form, the tails formed from the ends of the separate, spaced apart malleable wires could be adapted to project into the lumen of the first tubular graft body such that they engage with the second tubular graft body, thereby securing the second tubular graft body within the lumen of the first tubular graft body.

Also described herein, the first tubular body comprises a simple tubular sheath adapted to be disposed in a vessel such that it engages with and is attached to the wall of the vessel.

In a further configuration, the at least second tubular graft is preferably formed of a more durable and thick material than that of the first tubular graft body, for example Dacron ^{®} or polytetrafluoroethylene (PTFE).

In yet a further configuration, the surfaces of both the first and the second tubular graft bodies are coated with a material from the group comprising biocompatible glues, adhesives, or the like engineered cellular matrices for joining surfaces.. The biocompatible glue or the like adhesion means enhances attachment of the first tubular graft body to a vessel wall thereby further preventing dislodgement of the intraluminal device within the vessel and further enhancing attachment of the second tubular graft body to the first tubular graft body.

In another configuration, the surfaces of both the first and second tubular graft bodies may be coated with fibrins or some other material to stimulate fibrin or cellular ingrowth into the device from the surrounding tissue. Such ingrowth further secures the intraluminal device within the vessel wall. An example of a material which increases tissue ingrowth into the tubular graft bodies is polyurethane. Alternatively, a polyurethane/polycarbonate composite may be used to enhance cellular ingrowth.

Also described herein, the first and at least one second tubular graft bodies include a collar member attached to the first end. In addition, a further collar member may be attached to the at least one second end of the tubular graft bodies.

Alternatively, the collar member is not attached to the tubular graft bodies but is inserted separately from the tubular graft bodies.

The surface of the collar member may be coated with fibrins or some other material such as polyurethane or polyurethane/polycarbonate composite and adapted to stimulate cellular ingrowth into the device from the surrounding tissue.

In a further configuration, the first tubular graft body is circumferentially reinforced by a series of separate, spaced apart malleable wires along only a portion of its length.

In alternately, yet still another embodiment, the second tubular graft body is circumferentially reinforced by a series of separate, spaced apart malleable wires along only a portion of its length, see for example US 6 071 307.

In a further configuration, when the device is *in situ* within the vessel of a patient, the portion of the first tubular graft body that is not reinforced overlaps with the portion of the second tubular graft body that is reinforced and the portion of the second tubular graft body that is not reinforced overlaps with the portion of the first tubular graft body that is reinforced. In this case, the entire length of the device will be reinforced.

When the device is *in situ* within a vessel of a patient, only a superior portion of the first tubular graft body distal its own entry point is circumferentially reinforced by the wires and only an inferior portion of the second tubular graft body proximal its entry point is reinforced by the wires such that the overlapping of first and second tubular graft bodies causes the entire length of the device to be circumferentially reinforced. The circumferential reinforcement of each tubular graft body is not, however, limited to circumferential reinforcement of the superior or inferior portions of each tubular graft body and is simply adapted such that *in situ*, a length of the device that is not reinforced by the separate spaced apart malleable wires located on the first tubular graft body is reinforced by separate, spaced apart, malleable wires located on the second tubular graft body.

The intraluminal device may be used to treat aneurysmal or occlusive disease. In addition to treating aortic aneurysms they are particularly suitable for treating aneurysms of the femoral artery, the popliteal artery, the thoracic segment of the aorta, the visceral arteries such as the renal and mesenteric arteries, the iliac and subclavian artery.

It is sometimes the case that the aneurysm extends to or slightly beyond an arterial bifurcation. In such a case, the second tubular graft body is adapted such that it is bifurcated at its downstream end, a so-called "trouser graft". A supplemental graft, or cuff-means may then be introduced through each of the subsidiary arteries and overlapped with the respective lumenae of the bifurcated part of the second tubular graft body. For instance, in the case of an aneurysm in the aorta that extends into one or each of the iliac arteries, the second tubular graft body would be placed in the aorta through one of the iliac arteries. Supplemental grafts which dock with the bifurcated end of the primary graft would then be inserted through each of the iliac arteries. In such a case the region of graft body directly superior the bifurcated region of the second tubular graft body can have a propensity to occasionally kink. The overlapping of the first and second tubular graft bodies, therefore, increases the circumferential reinforcement in the region of the device superior the bifurcated region by increasing the number of separate, spaced apart, malleable wires or other reinforcing wires thereby reducing the likelihood of kinking in this region. In an embodiment, the first and at least second tubular graft bodies are longitudinally reinforced in addition to or instead of being circumferentially reinforced.

In a further embodiment of the invention, the second tubular graft body is inserted into the lumen of the first tubular graft body such that a substantial length of the second tubular graft body overlaps with a substantial length of the first tubular graft body.

In still a further embodiment of this aspect of the invention, the second tubular graft body is of the "trouser graft" type such that the bifurcated portion is positioned such that it extends longitudinally from the first tubular graft body into the surrounding vessel, for example, the aorta and wherein a supplemental graft may be introduced though each of the subsidiary arteries and overlapped with the respective lumenae of the bifurcated portion of the second tubular graft body.

In another embodiment, where the intraluminal device is adapted to span an aneurysm affecting an area of artery which is bifurcated, both the tubular graft bodies are of the "trouser graft" variety. Accordingly, the tubular graft bodies comprise a main body positioned in, for example, the aorta and two leg members adapted to extend into the iliac arteries. The first leg member of the first tubular graft body may be shorter than the second leg member. In this case, the second leg member of the second tubular graft member is shorter than the first leg member of the second tubular graft member. Accordingly, the two tubular graft members may be mirror images of each other or as close to mirror images as is practicable. This has the advantage of avoiding unnecessary bulking in the leg members of the graft due to excessive overlapping of the two grafts in this area. Because the main bodies of each tubular graft body overlap with each other, however, the intraluminal device is still reinforced in the area of the device most likely to kink, that is, the area directly above the bifurcation of the tubular graft bodies.

Also described herein, the first tubular graft body may include only one leg member, the first tubular graft body having an aperture rather than a second leg member. In this case, the second tubular member is adapted such that it has one leg member that may be inserted through the aperture of the first tubular graft body. In place of the second leg member, the second tubular member has an aperture through which the leg member of the first tubular graft body may be inserted. This feature has the advantage that each tubular graft member is symmetrical in shape. Typically, with trouser grafts, the tubular graft body must be positioned in a certain orientation, such that one leg member extends into one vessel and the other leg member extends into another vessel. In the case of a graft for bridging an aneurysm spanning the bifurcation of the aorta into the iliac arteries, one of the leg members will extend towards or into the left iliac artery and the other leg member will extend towards or into the right iliac artery. Radio-opaque markers positioned on the graft are typically used to ensure the correct positioning of the graft. However, because the first tubular graft body is symmetrical in shape there is no need to use such markers to ensure correct positioning of the tubular graft body and the one leg member will extend towards or into the desired iliac artery. A second tubular graft member may then be inserted, the second tubular graft member having one leg member that extends towards or into the other iliac artery.

According to a feature of the present invention, there is provided in an endovascularly emplaced prosthesis for bridging an aneurysm, the improvement which comprises; at least a supplemental graft member positioned whereby a flow path through a treated vessel is extended.

According to another feature of the present invention, there is provided, an apparatus for intraluminal emplacement comprising; a first tubular body; and a second tubular body wherein each said tubular body further comprises a graft having a length and a first and at least a second end, whereby when the apparatus is disposed within a vessel of a patient, a predetermined length of the second graft body overlaps with a desired length of the first graph body.

Also described herein, there is provided a method of intraluminal emplacement comprising:
providing a first graft body and positioning a cuff-means for extending the first graft body within said first graft body for affixing said cuff-means whereby a lumen of said first graft body is extended.

### Brief Description of the Drawings

Figure 1 is a diagrammatic partially cut-away ventral view of a patient with an aortic aneurysm which has been bridged by an intraluminal graft.

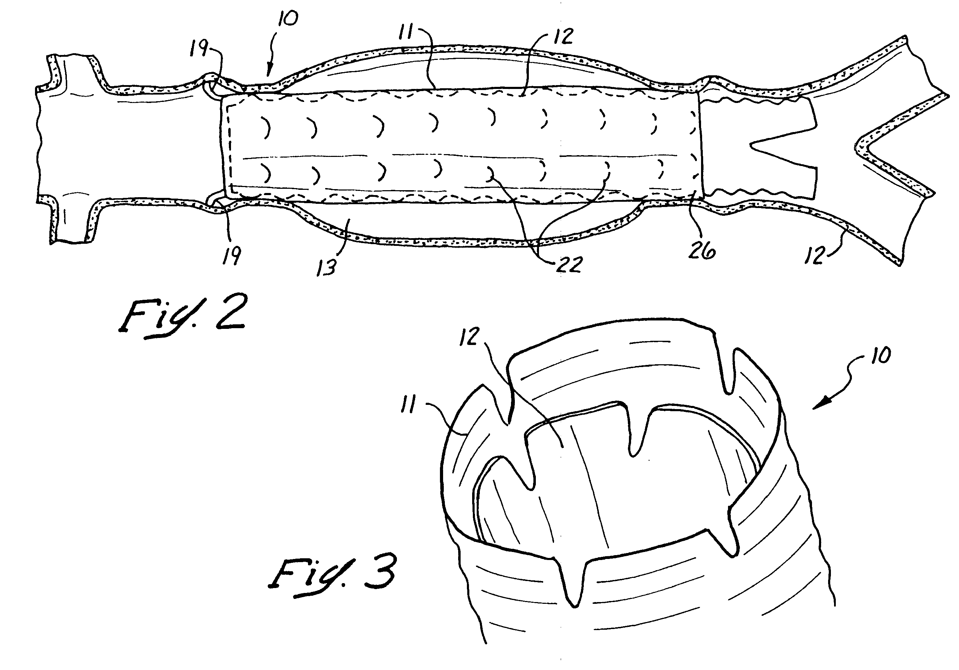

Figure 2 is a detailed longitudinal sectional view of the intraluminal device of Figure 1.
Figure 3 is a detailed elevational view of one end of an intraluminal device.
Figure 4 is a detailed view of one component of an intraluminal device.
Figure 5 is a detailed view of one component of an intraluminal device.

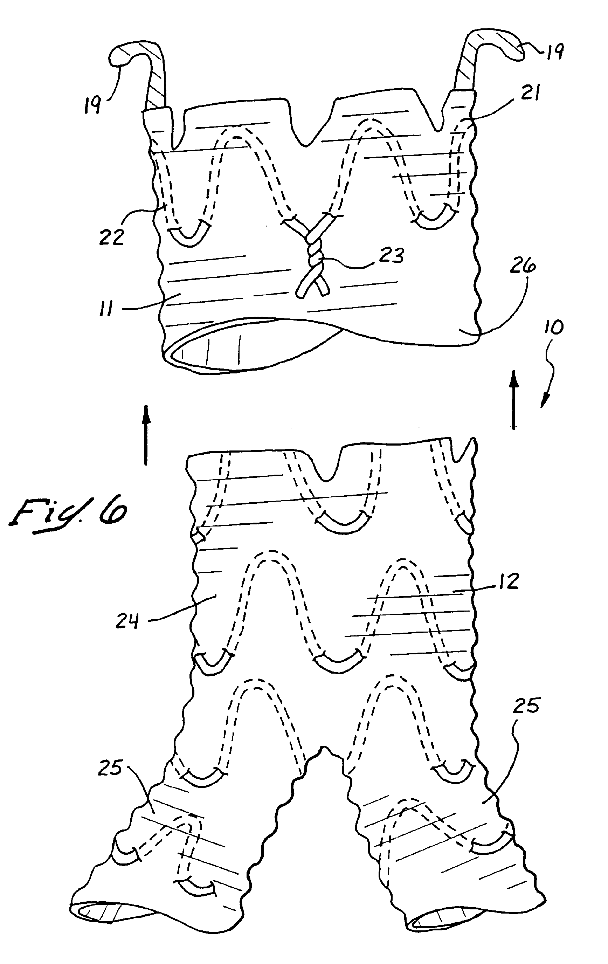

Figure 6 is a side elevational view of an intraluminal device showing the spatial arrangement between the components of the device.
Figure 7 is a detailed view of one component of intraluminal device.
Figure 8 is a side elevational view of one embodiment of the invention.
Figure 9 is a side elevational view of one component of the embodiment of the invention depicted in Figure 8.
Figure 10 is a side elevational view of another component of the embodiment of the invention depicted in Figure 8.
Figure 11 is a more detailed side elevational view of the embodiment of the invention as depicted in Figure 8.
Figure 12 is a schematic view not of the current invention.
Figure 13 is a side elevational view of not of the current invention.
Figure 14 shows a supplemental cuff-means not of the current invention.

### Detailed Description of the Drawings

An intraluminal device according to the present invention is generally shown as 10 in the drawings. The intraluminal device 10 comprises two separate components, a first graft 11 and a second graft 12.

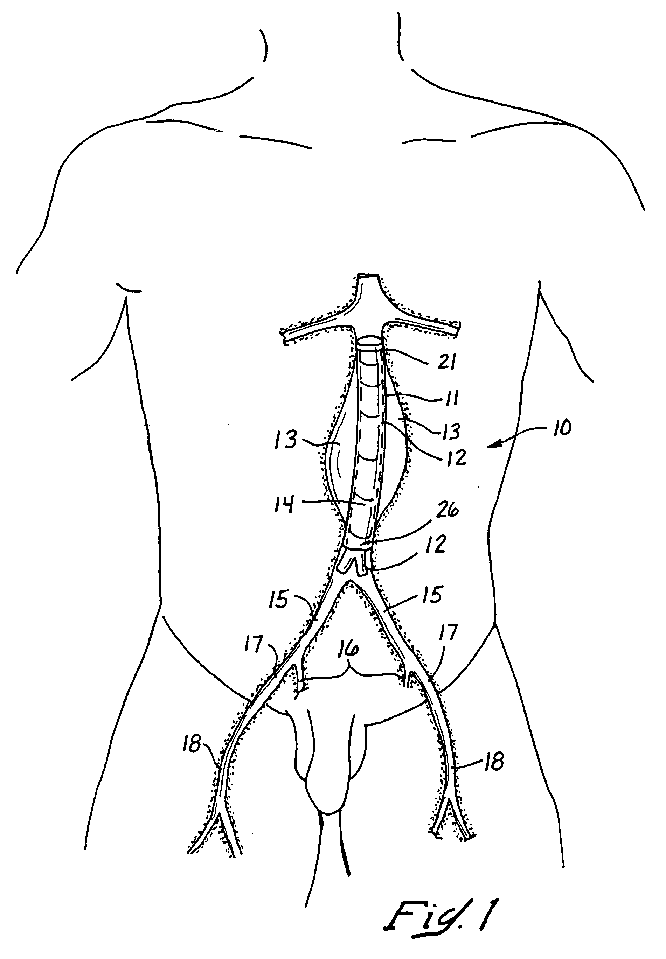

The device 10 is adapted for insertion transfemorally into a patient to achieve bridging and occlusion of an aneurysm 13 present in the aorta 14. As shown in Fig 1, the aorta 14 bifurcates to form the common iliac arteries 15 which in turn divide into the internal 16 and external 17 iliac arteries. The external iliac artery in turn forms the femoral artery 18. The first graft 11 is inserted inside a catheter (not shown) and introduced into one of the femoral arteries 18 in a leg of a patient. Once the catheter is located appropriately with its proximal end in the aorta 14, the first graft 11 is ejected from the catheter and expanded using a balloon so that the first graft 11 is in intimate contact along its length and around its full periphery with the surrounding vessel. The first graft 11 then bridges the aneurysm. Alternatively, in a preferred embodiment, the first graft 11 is made from a self expandable material such that first graft 11 is in a collapsed configuration internal the catheter. Upon ejection from the catheter, the first graft takes on an expanded configuration such that the first graft 11 is in intimate contact along its length and around its full periphery with the surrounding vessel.

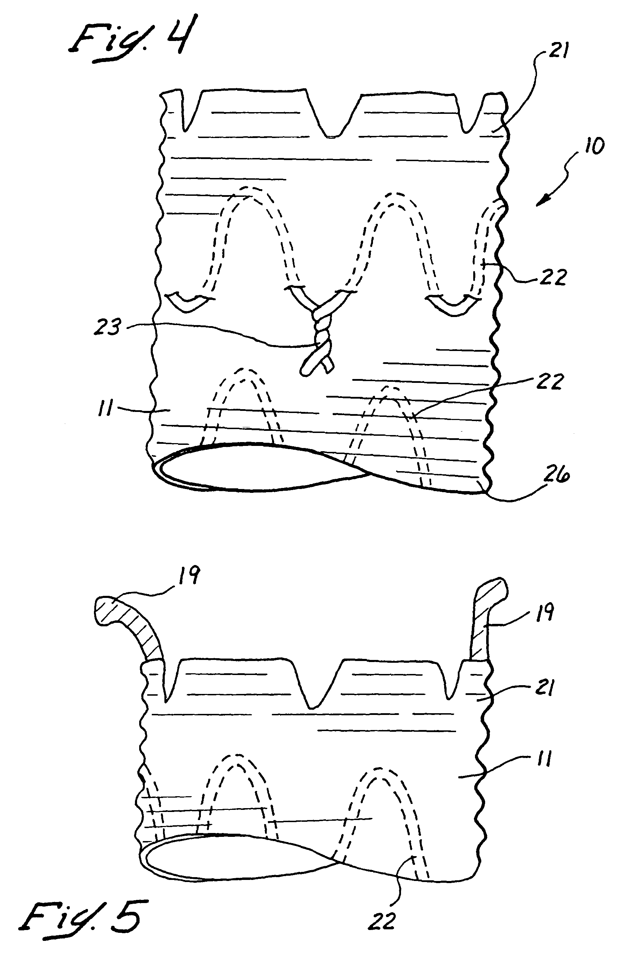

The first graft 11 comprises a tube made from a very thin material and is used essentially as an anchor for the second more durable graft 12. To assist in the placement of the first graft 11 in the aorta 14, the first graft 11 is provided with engagement members 19 (see Fig. 5) which project from one end 21 of the first graft 11. It is to be recognised that further engagement members may be circumferentially disposed along the entire length of the first graft 11.

The first graft 11 and second graft 12 may be circumferentially reinforced along their lengths by a number of separate and spaced wires 22. The wires are preferably as thin as possible and are malleable such that they may be bent to any desired shape. The wires are each woven into the fabric of the first graft 11 or second graft 12 such that alternate crests of each wire 22 are outside of the graft with the remainder of the wire 22 inside the graft. The ends of each wire 22 are located outside the first graft 11 or second graft 12 and are twisted together to form a tail 23. The tails or alternate wires may be bent in opposite longitudinal directions along the outside of the surface of the first graft 11. The arrangement of the tails 23 on the outside of the first graft 11 assists in the positioning of the first graft 11 in the aorta as the tails 23 have a tendency to abut against the wall of the aorta thereby securing the first graft 11 within the aorta.

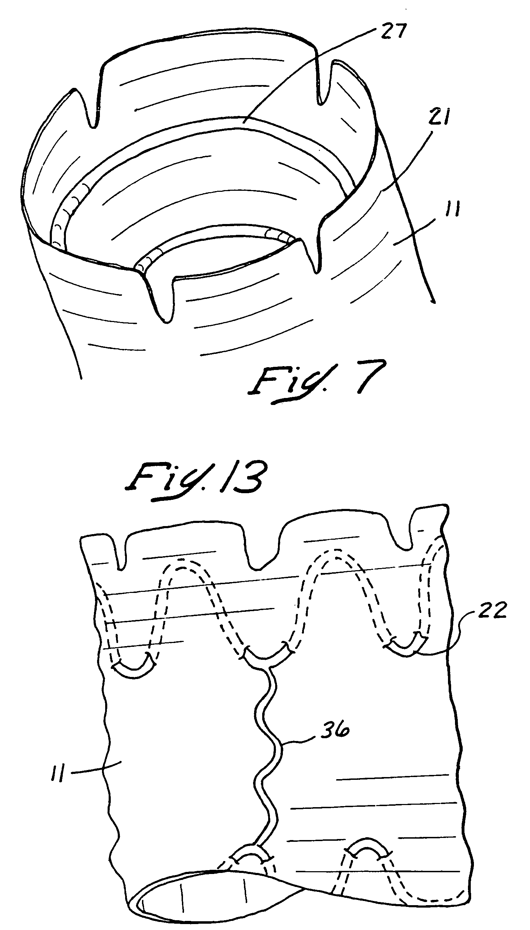

The first graft 11 and the second graft 12 may be longitudinally reinforced by wire 36. Figure 13 depicts the longitudinal reinforcement of the first graft 11 only but it is readily envisaged that second graft 12 may be similarly reinforced. Further, wire 36 is shown as being connected to the circumferentially reinforcing wires 22. It is to be understood that wire 36 may be connected to the material of the first graft 11 and not to the wires 22. Wire 36 may be straight or zig-zag in shape or may be a sinusoidal shape as depicted in Figure 13.

Once the first graft 11 is in position, the second graft 12 is similarly introduced into the aorta 14 by way of insertion of a catheter through the femoral artery 18 of a patient. In the depicted embodiment, the second graft 12 is of the "trouser graft" type, that is, it has a main body 24 and a bifurcated portion 25 and is made from woven Dacron^{®} The catheter is introduced into the lumen of the first graft 11 and the second graft 12 inflated by way of a balloon such that the main body 24 expands and abuts against the inner facing surface of the first graft 11. The bifurcated portion 25 extends in a longitudinal plane from the other end 26 of the first graft 11 into the lumen of the aorta 14.

The first graft 11 may be provided with internal rings 27 which act to overlay the wires 22 of the second graft 12 and thereby secure the second graft 12 in place within the lumen of the first graft 11.

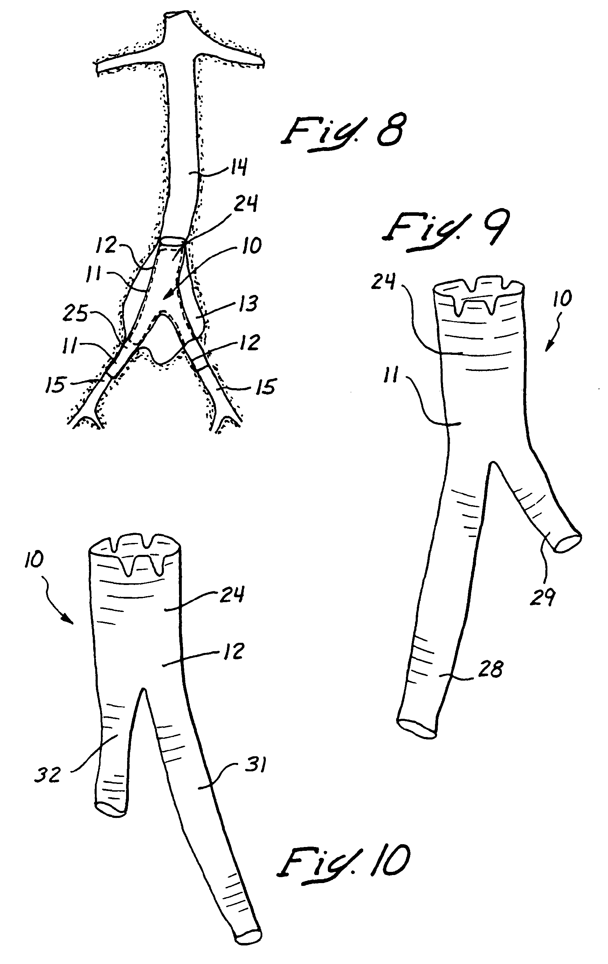

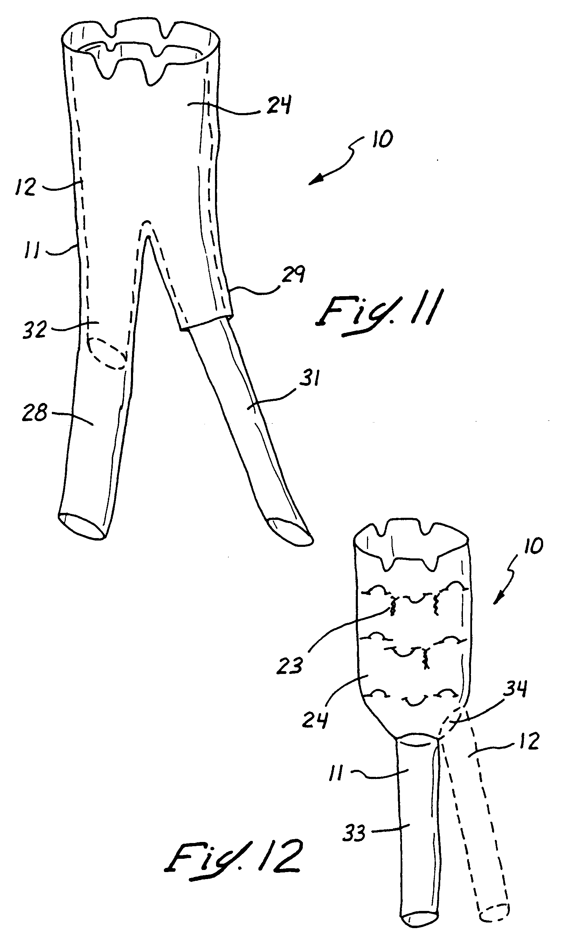

Figure 8 to 10 depict the embodiment of the invention wherein both graft 11 and 12 are "trouser grafts". The first graft 11 has a main body 24, a long leg 28 and a short leg 29. The second graft 12 also has a main body 24, a long leg 31 and a short leg 32. When the two grafts are in situ within a vessel of a patient, as depicted in Figure 11, the main body 24 of each graft overlap with each other entirely whereas overlapping of the legs of the grafts is kept to a minimum to avoid unnecessary "bulking" of the graft in this area.

Also described herein and depicted in Figure 12, the first graft 11 has a main body 24 and one leg 33. A second leg is absent in its place is simply an aperture 34. A second graft 12 may be inserted internal the first graft 11 such a leg 35 of the second graft 12 extends through aperture 34. The leg 35 of second graft 12 extending through aperture 34 is shown in phantom in Figure 12.

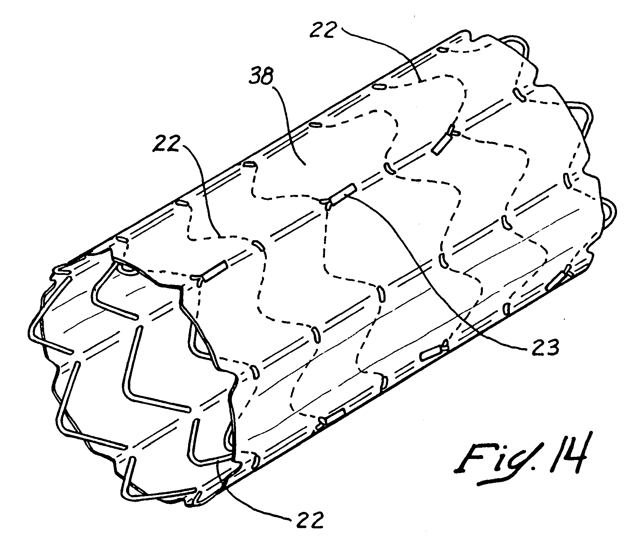

Referring now to Fig. 14, supplemental cuff-means 38 likewise comprises a plurality of crimped stent-formed wires 22 which are each woven into the fabric of supplemental graft 38 such that alternate crests of each wire 22 are outside of graft 38 with the remainder of the wire 22 inside the graft. The ends of each wire 22 are located outside graft 38 and are twisted together to form a tail 23. The tails or alternate wires may be bent in opposite longitudinal directions along the outside of the surface of graft 38. The arrangement of the tails 23 on the outside of graft 38 assists in the positioning of graft 38 in the aorta as the tails 23 have a tendency to abut against the wall of the aorta thereby securing graft 38 within the aorta.

Likewise, those having a modicum of skill in the art will understand that supplemental cuff-means or graft 38 is effective for use within the (failed or leaking) grafts of other commercial entities, such as, for example, the GUIDANT/EVT brand ANCURB® DEVICE; the GORE brand EXCLUDER®; the Boston Scientific Vanguard® brand; Medtronic/AVE ANERUERX® or TALENT ® brand devices in addition to those of the Cook Endovascular Graft brandZENITH™ AAA Endovascular Graft. It is noted that specific overlapping, sizing, and the like dimensional parameters will be obvious to artisans.

## Claims

1. An intraluminal device (10), comprising:
a first bifurcated graft (11) comprising a first main tubular body portion (24) having a length, a first leg (28) having a first leg length and a second leg (29) having a second leg length which is shorter than the first leg length; and
a second bifurcated graft (12) comprising a second main tubular body portion (24) having a length, a first leg (31) having a first leg length and a second leg (32) having a second leg length which is shorter than the first leg length;
wherein when the intraluminal device (10) is disposed within a vessel of a patient, a majority of the length of the first main tubular body portion (24) overlaps with a majority of the length of the second main tubular body portion (24), the first leg of the first bifurcated graft (28) extends through the second leg of the second bifurcated graft (32), and the second leg of the first bifurcated graft (29) extends through the first leg of the second bifurcated graft (31).

2. The device of claim 1 wherein at least a part of the length of one of the first (11) and the second (12) bifurcated grafts is balloon expandable while the remaining length of the same bifurcated graft is self-expanding.

3. The device of claim 1 wherein one of the first (11) and second (12) bifurcated grafts is balloon expandable and the other of the first (11) and second (12) bifurcated grafts is seu-expanding.

4. The device of claim 1 wherein at least one of the first (11) and the second (12) bifurcated grafts is reinforced along its length by a plurality of separate spaced apart wires (22).

5. The device of claim 4 wherein a proximal portion of one of the first (11) and the second (12) bifurcated grafts is circumferentially reinforced by a plurality of separate spaced apart wires (22).

6. The device of claim 1 wherein the first (11) and second (12) bifurcated grafts are capable of an overlap which is greater than 75% of the length of one of the first (11) and second (12) bifurcated grafts.

7. The device of claim 6 wherein the entire length of one of the first (11) and second (12) bifurcated grafts can overlap with the other of the first (11) and second bifurcated (12) grafts.

8. The device of claim 7 wherein the first (11) and the second (12) bifurcated grafts have substantially the same length.

9. The device of claim 1 wherein the first (11) and the second (12) bifurcated grafts are reinforced along their lengths by a continuous wire of a spiral configuration (22).

10. The device of claim 1 wherein the second bifurcated is grafts (12) formed of a more durable material than the first bifurcated graft (11).

11. The device of claim 1 wherein the first bifurcated graft (11) is graft (11) formed of a thinner material than the second bifurcated graft (12).

12. The device of claim 1 wherein a surface of at least one of the first (11) and second (12) bifurcated grafts is coated with material that stimulates fibrin or cellular ingrowth into the device from the surrounding tissue to secure the device within the vessel of the patient

13. The device of claim 1 wherein the surfaces of both the first (11) and second (12) bifurcated grafts are coated with a material selected from glues, adhesives and cellular matrices, to enhance attachment of the first and second bifurcated grafts.

## Patentansprüche

1. Intraluminale Einrichtung (10) mit:
einem ersten verzweigten Transplantat (11), welches einen ersten rohrförmigen Hauptkörperbereich (24) mit einer Länge, ein erstes Bein (28) mit einer ersten Beinlänge und ein zweites Bein (29) mit einer zweiten Beinlänge, die kleiner ist als die erste Beinlänge, aufweist; und
einem zweiten verzweigten Transplantat (12), welches einen zweiten rohrförmigen Hauptkörperbereich (24) mit einer Länge, ein erstes Bein (31) mit einer ersten Beinlänge und ein zweites Bein (32) mit einer zweiten Beinlänge, die kleiner ist als die erste Beinlänge, aufweist,
wobei bei Anordnung der intraluminalen Einrichtung (10) in einem Gefäß eines Patienten ein Großteil der Länge des ersten rohrförmigen Hauptkörperbereichs (24) überlappt mit einem Großteil der Länge des zweiten rohrförmigen Hauptkörperbereichs (24), sich das erste Bein des ersten verzweigen Transplantates (28) durch das zweite Bein des zweiten verzweigten Transplantates (32) erstreckt und sich das zweite Bein des ersten verzweigten Transplantates (29) durch das erste Bein des zweiten verzweigten Transplantates (31) erstreckt.

2. Einrichtung nach Anspruch 1, **dadurch gekennzeichnet, dass** zumindest ein Teil der Länge des ersten verzweigten Transplantats (11) oder des zweiten verzweigten Transplantats (12) mit einem Ballon expandierbar ist, während ein verbleibender Längsabschnitt dieses verzweigten Transplantates selbstexpandierend ausgebildet ist.

3. Einrichtung nach Anspruch 1, **dadurch gekennzeichnet, dass** das erste verzweigte Transplantat (11) oder das zweite verzweigte Transplantat (12) mit einem Ballon expandierbar ist und das andere verzweigte Transplantat (12, 11) selbstexpandierend ausgebildet ist.

4. Einrichtung nach Anspruch 1, **dadurch gekennzeichnet, dass** das erste verzweigte Transplantat (11) und/oder das zweite verzweigte Transplantat (12) entlang der Länge verstärkt ist durch mehrere separate beabstandete Drähte (22).

5. Einrichtung nach Anspruch 4, **dadurch gekennzeichnet, dass** ein proximaler Bereich des ersten verzweigten Transplantats (11) oder des zweiten verzweigten Transplantats (12) in Umfangsrichtung verstärkt ist durch mehrere separate beabstandete Drähte (22).

6. Einrichtung nach Anspruch 1, **dadurch gekennzeichnet, dass** das erste verzweigte Transplantat (11) und das zweite verzweigte Transplantat (12) geeignet ausgebildet sind zur Bildung einer Überlappung, die größer ist als 75 % der Länge des ersten verzweigten Transplantats (11) oder des zweiten verzweigten Transplantats (12).

7. Einrichtung nach Anspruch 6, **dadurch gekennzeichnet, dass** die gesamte Länge des ersten verzweigten Transplantats (11) oder des zweiten verzweigten Transplantats (12) mit dem anderen Transplantat (12; 11) überlappen kann.

8. Einrichtung nach Anspruch 7, **dadurch gekennzeichnet, dass** das erste und zweite verzweigte Transplantat (11, 12) substantiell oder ungefähr dieselbe Länge besitzen.

9. Einrichtung nach Anspruch 1, **dadurch gekennzeichnet, dass** das erste und zweite verzweigte Transplantat (11, 12) entlang ihrer Längen durch einen kontinuierlichen spiralförmigen Draht (22) verstärkt sind.

10. Einrichtung nach Anspruch 1, **dadurch gekennzeichnet, dass** das zweite verzweigte Transplantat (12) aus einem widerstandsfähigeren oder beständigeren Material gebildet ist als das erste verzweigte Transplantat (11).

11. Einrichtung nach Anspruch 1, **dadurch gekennzeichnet, dass** das erste verzweigte Transplantat (11) aus einem dünneren Material gebildet ist als das zweite verzweigte Transplantat (12).

12. Einrichtung nach Anspruch 1, **dadurch gekennzeichnet, dass** eine Oberfläche oder die Oberflächen des ersten verzweigten Transplantats (11) und/oder des zweiten verzweigten Transplantats (12) überzogen oder ummantelt ist oder sind mit einem Material, welches ein Einwachsen von Fibrin oder ein zellenartiges Einwachsen in die Einrichtung von umgebendem Gewebe stimuliert, um die Einrichtung in dem Gefäß des Patienten zu befestigen oder zu sichern.

13. Einrichtung nach Anspruch 1, **dadurch gekennzeichnet, dass** die Oberflächen des ersten verzweigten Transplantats (11) und des zweiten verzweigten Transplantats (12) mit einem der Materialen Leim, ein adhäsives Mittel oder zellförmige Matrizen ummantelt oder überdeckt sind, um die Befestigung des ersten und zweiten verzweigten Transplantats zu verbessern.

## Revendications

1. Dispositif intraluminal (10) comprenant :
une première greffe bifurquée (11) comprenant une première partie de corps tubulaire principal (24) ayant une longueur, une première patte (28) ayant une première longueur de patte et une seconde patte (29) ayant une seconde longueur de patte qui est plus courte que la première longueur de patte ; et
une seconde greffe bifurquée (12) comprenant une seconde partie de corps tubulaire principal (24) ayant une longueur, une première patte (31) ayant une première longueur de patte et une seconde patte (32) ayant une seconde longueur de patte qui est plus courte que la première longueur de patte ;
dans lequel lorsque le dispositif intraluminal (10) est disposé à l'intérieur d'un vaisseau d'un patient, une majeure partie de la longueur de la première partie de corps tubulaire principal (24) chevauche sur une majeure partie de la longueur de la seconde partie de corps tubulaire principal (24), la première patte de la première greffe bifurquée (28) s'étend à travers la seconde patte de la seconde greffe bifurquée (32) et la seconde patte de la première greffe bifurquée (29) s'étend à travers la première patte de la seconde greffe bifurquée (31).

2. Dispositif selon la revendication 1, dans lequel au moins une partie de la longueur de l'une parmi la première (11) et la seconde (12) greffe bifurquée est un ballonnet expansible alors que la longueur restante de la même greffe bifurquée est autoexpansible.

3. Dispositif selon la revendication 1, dans lequel l'une parmi la première (11) et la seconde (12) greffe bifurquée est un ballonnet expansible et l'autre parmi la première (11) et la seconde (12) greffe bifurquée est autoexpansible.

4. Dispositif selon la revendication 1, dans lequel au moins l'une parmi la première (11) et la seconde (12) greffe bifurquée est renforcée le long de sa longueur par une pluralité de fils (22) séparés espacés.

5. Dispositif selon la revendication 4, dans lequel une partie proximale de l'une parmi la première (11) et la seconde (12) greffe bifurquée est renforcée de manière circonférentielle par une pluralité de fils (22) séparés espacés.

6. Dispositif selon la revendication 1, dans lequel la première (11) et la seconde (12) greffe bifurquée peuvent réaliser un chevauchement qui est supérieur à 75% de la longueur de l'une parmi la première (11) et la seconde (12) greffe bifurquée.

7. Dispositif selon la revendication 6, dans lequel toute la longueur de l'une parmi la première (11) et la seconde (12) greffe bifurquée peut chevaucher sur l'autre parmi la première (11) et la seconde (12) greffe bifurquée.

8. Dispositif selon la revendication 7, dans lequel la première (11) et la seconde (12) greffe bifurquée ont sensiblement la même longueur.

9. Dispositif selon la revendication 1, dans lequel la première (11) et la seconde (12) greffe bifurquée sont renforcées le long de leurs longueurs par un fil continu ayant une configuration en spirale (22).

10. Dispositif selon la revendication 1, dans lequel la seconde greffe bifurquée (12) est formée avec un matériau plus durable que la première greffe bifurquée (11).

11. Dispositif selon la revendication 1, dans lequel la première greffe bifurquée (11) est formée avec un matériau plus fin que la seconde greffe bifurquée (12).

12. Dispositif selon la revendication 1, dans lequel une surface d'au moins l'une parmi la première (11) et la seconde (12) greffe bifurquée est recouverte avec un matériau qui stimule la fibrine ou la croissance cellulaire dans le dispositif du tissu environnant pour fixer le dispositif à l'intérieur du vaisseau du patient.

13. Dispositif selon la revendication 1, dans lequel les surfaces à la fois de la première (11) et de la seconde (12) greffe bifurquée sont recouvertes avec un matériau choisi parmi les colles, les adhésifs et les matrices cellulaires afin d'améliorer la fixation des première et seconde greffes bifurquées.
